(19) Europäisches Patentamt
European Patent Office
Office européen des brevets

(11) Veröffentlichungsnummer: **0 000 887**
**B1**

(12) # EUROPÄISCHE PATENTSCHRIFT

(21) Anmeldenummer: **78100599.6**

(22) Anmeldetag: **07.08.78**

(51) Int. Cl.³: **C 07 F 9/65**, A 01 N 57/16, A 01 N 57/18, A 01 N 57/28

(54) **Cyclopropylsubstituierte Pyrimidin(4)yl(thiono)(thiol)-phosphor(phosphon)-säureester bzw. -esteramide, Verfahren zu ihrer Herstellung und ihre Verwendung als Insektizide und Akarizide**

(30) Priorität: **19.08.77 DE 2737401**

(43) Veröffentlichungstag der Anmeldung:
**07.03.79 Patentblatt 79/05**

(45) Bekanntmachung des Hinweises auf die Patenterteilung:
**29.10.80 Patentblatt 80/22**

(84) Benannte Vertragsstaaten:
**BE CH DE FR GB NL**

(56) Entgegenhaltungen:
**DE - A - 2 209 554**
**DE - A - 2 412 903**
**US - A - 2 754 243**
**US - A - 4 012 506**

(73) Patentinhaber: **BAYER AG**
**Zentralbereich Patente, Marken und Lizenzen**
**D - 5090 Leverkusen 1, Bayerwerk (DE)**

(72) Erfinder: **Maurer, Fritz, Dr.**
**Roeberstrasse 8**
**D - 5600 Wuppertal 1 (DE)**
**Hammann, Ingeborg, Dr.**
**Belfortstrasse 9**
**D - 5000 Köln (DE)**
**Behrenz, Wolfgang, Dr.**
**Untergründemich 14**
**D - 5063 Overath (DE)**
**Stendel, Wilhelm, Dr.**
**In den Birken 55**
**D - 5600 Wuppertal 1 (DE)**

**EP 0 000 887 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Cyclopropylsubstituierte Pyrimidin(4)yl(thiono)(thiol)-phosphor(phosphon)-säureester bzw. -esteramide, Verfahren zu ihrer Herstellung und ihre Verwendung als Insektizide und Akarizide

Die vorliegende Erfindung betrifft neue cyclopropylsubstituierte Pyrimidin(4)yl(thiono)(thiol)-phosphor(phosphon)-säureester bzw. -esteramide, welche insektizide und akarizide Eigenschaften besitzen sowie ein Verfahren zu ihrer Herstellung.

Es ist bereits bekannt, daß O,O-Dialkyl-O-pyrimidinylthionophosphorsäureester, z.B. O,O-Dimethyl-O-[2-äthyl-4-äthoxypyrimidin(6)yl]- bzw. O,O-Diäthyl-O-[2-isopropyl-4-methyl-pyrimidin(6)yl]-thionophosphorsäureester, insektizide und akarizide Eigenschaften haben (vergleiche US-Patentschriften 2 754 243 und 3 862 188).

Außerdem sind aus der US—PS 4.012.506 cyclopropylsubstituierte Pyrimidin-(4)-yl-thio- und dithiophosphorsäureester mit insektizider und akarizider Wirksamkeit bekannt. Diese Wirkstoffe sind jedoch, wie entsprechende Vergleichsversuche zeigen, den erfindungsgemäßen Verbindungen in der insektiziden und akariziden Wirksamkeit unterlegen.

Es wurden nun die neuen cyclopropylsubstituierten Pyrimidin-(4)-yl(thiono)(thiol)-phosphor(phosphon)-säureester bzw. -esteramide der Formel

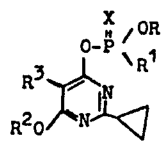

synthetisiert,
in welcher
R für Alkyl,
$R^1$ für Alkyl, Alkoxy, Alkylthio, Monoalkylamino oder Phenyl,
$R^2$ für Alkyl,
$R^3$ für Wasserstoff, Halogen oder Alkyl und
X für Sauerstoff oder Schwefel stehen.

Diese neuen Verbindungen besitzen eine ausgezeichnete insektizide und akarizide Wirkung.

Weiterhin wurde gefunden, daß man die cyclopropylsubstituierten Pyrimidin(4)yl(thiono)(thiol)-phosphor(phosphon)-säureester bzw. -esteramide der Formel (I) erhält, wenn man (Thiono)(Thiol)-Phosphor(phosphon)-säureester- bzw. -esteramidhalogenide der Formel

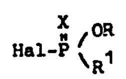 (II)

in welcher
R, $R^1$ und X die oben angegebene Bedeutung haben und
Hal für Halogen, vorzugsweise Chlor, steht, mit 2-Cyclopropyl-4-hydroxy-pyrimidinen der Formel

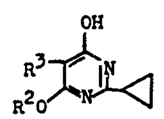 (III)

in welcher
$R^2$ und $R^3$ die oben angegebene Bedeutung haben, gegebenenfalls in Gegenwart eines Säureakzeptors oder gegebenenfalls in Form der Alkali-, Erdalkali- oder Ammoniumsalze und gegebenenfalls in Gegenwart eines Lösungs- oder Verdünnungsmittels umsetzt.

Überraschenderweise zeigen die erfindungsgemäßen cyclopropylsubstituierten Pyrimidin(4)yl(thiono)(thiol)-phosphor(phosphon)-säureester bzw. -esteramide eine bessere insektizide und akarizide Wirkung als die bekannten Pyrimidin(6)yl-thionophosphorsäureester analoger Konstitution und gleicher Wirkungsrichtung. Die Verbindungen vorliegender Erfindung stellen somit eine echte Bereicherung der Technik dar.

Verwendet man beispielsweise O-Äthyl-S-n-propyl-thionothiolphosphorsäurediesterchlorid und 2-Cyclopropyl-5-methyl-6-isopropyloxy-4-hydroxy-pyrimidin als Ausgangsstoffe, so kann der Reaktionsverlauf durch das folgende Formelschema wiedergegeben werden:

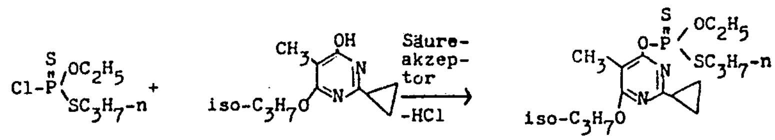


Die zu verwendenden Ausgangsstoffe sind durch die Formeln (II) und (III) allgemein definiert. Vorzugsweise stehen darin jedoch

R für geradkettiges oder verzweigtes Alkyl mit 1 bis 6, insbesondere 1 bis 4, Kohlenstoffatomen,
$R^1$ für geradkettiges oder verzweigtes Alkyl bzw. Alkoxy, Alkylthio oder Monoalkylamino mit 1 bis 6 insbesondere 1 bis 4, Kohlenstoffatomen je Alkylrest oder Phenyl,
$R^2$ für geradkettiges oder verzweigtes Alkyl mit 1 bis 6, insbesondere 1 bis 4, Kohlenstoffatomen,
$R^3$ für Wasserstoff, Chlor, Brom, oder geradkettiges oder verzweigtes Alkyl mit 1 bis 5 Kohlenstoffatomen, insbesondere Methyl oder Äthyl, und
X für Schwefel.

Die als Ausgangsstoffe zu verwendenden (Thiono)(Thiol)Phosphor(phosphon)-säureester-bzw -esteramidhalogenide (II) sind bekannt und nach literaturbekannten Verfahren auch technisch gut herstellbar. Als Beispiele dafür seien im einzelnen genannt: O,O-Dimethyl-, O,O-Diäthyl-, O,O-Di-n-propyl-, O,O-Di-iso-propyl-, O,O-Di-n-butyl-, O,O-Di-iso-butyl-, O,O-Di-sek.-butyl-, O-Methyl-O-äthyl-, O-Methyl-O-n-propyl-, O-Methyl-O-iso-propyl-, O-Methyl-O-n-butyl-, O-Methyl-O-iso-butyl-, O-Methyl-O-sek.-butyl-, O-Äthyl-O-n-propyl-, O-Äthyl-O-iso-propyl-, O-Äthyl-O-n-butyl-, O-Äthyl-O-sek.-butyl-, O-Äthyl-O-iso-butyl-, O-n-Propyl-O-butyl- bzw. O-iso-Propyl-O-butylphosphorsäurediesterchlorid und die entsprechenden Thionoanalogen, ferner O,S-Dimethyl-, O,S-Diäthyl-, O,S-Di-n-propyl-, O,S-Di-iso-propyl-, O,S-Di-n-butyl-, O,S-Di-iso-butyl-, O-Äthyl-S-n-propyl-, O-Äthyl-S-iso-propyl-, O-Äthyl-S-n-butyl-, O-Äthyl-S-sek.-butyl-, O-n-Propyl-S-äthyl-, O-n-Propyl-S-iso-propyl-, O-n-Butyl-S-n-propyl- und O-sek.-Butyl-S-äthylthiolphosphorsäurediesterchlorid und die entsprechenden Thionoanalogen, ferner O-Methyl-, O-Äthyl-, O-n-Propyl-, O-iso-Propyl-, O-n-Butyl-, O-iso-Butyl- bzw. O-sek.-Butyl-methan- bzw. -äthan-, -n-propan-, -iso-propan-, -n-butan-, -iso-butan-, -sek.-butan- bzw. -phenyl-phosphonsäureesterchlorid und die entsprechenden Thionoanalogen, und O,N-Dimethyl-, O-Methyl-N-äthyl-, O-Methyl-N-n-propyl-, O-Methyl-N-iso-propyl-, O-Äthyl-N-methyl-, O,N-Diäthyl-, O-Äthyl-N-n-propyl-, O-Äthyl-N-iso-propyl-, O-n-Propyl-N-methyl-, O-n-Propyl-N-äthyl-, O,N-Di-n-propyl-, O-n-Propyl-N-iso-propyl-, O-iso-Propyl-N-methyl-, O-iso-Propyl-N-äthyl-, O-iso-Propyl-N-n-propyl, O,N-Di-iso-propyl-, O-n-Butyl-N-methyl-, O-n-Butyl-N-äthyl-, O-n-Butyl-N-n-propyl-, O-n-Butyl-N-iso-propyl-, O-iso-Butyl-N-methyl-, O-iso-Butyl-N-äthyl-, O-iso-Butyl-N-n-propyl-, O-iso-Butyl-N-iso-propyl-, O-sek.-Butyl-N-methyl-, O-sek.-Butyl-N-äthyl-, O-sek.-Butyl-N-n-propyl- und O-sek.-Butyl-N-iso-propylphosphorsäuremonoesteramidchlorid und die entsprechenden Thionoanalogen.

Die weiterhin als Ausgangsstoffe zu verwendenden 2-Cyclopropyl-4-hydroxy-pyrimidine (III) können nach literaturbekannten Verfahren hergestellt werden, indem man z.B. 2-Cyclopropyl-4,6-dihydroxy-pyrimidin alkyliert, z.B. mit Dimethylsulfat, und gegebenenfalls die erhaltenen Produkte halogeniert, z.B. mit Brom.

Als Beispiele dafür seien im einzelnen genannt: 6-Methoxy-, 6-Äthoxy, 6-n-Propoxy-, 6-iso-Propoxy-, 6-n-Butoxy-, 6-sek.-Butoxy-, 6-iso-Butoxy- und 6-tert.-Butoxy-2-cyclopropyl-4-hydroxy-pyrimidin, ferner 5-Chlor-6-methoxy-, 5-Chlor-6-äthoxy-, 5-Chlor-6-n-propoxy-, 5-Chlor-6-iso-propoxy-, 5-Chlor-6-n-butoxy-, 5-Chlor-6-sek.-butoxy-, 5-Chlor-6-iso-butoxy- und 5-Chlor-6-tert.-butoxy-2-cyclopropyl-4-hydroxy-pyrimidin, ferner 5-Brom-6-methoxy- 5-Brom-6-äthoxy-, 5-Brom-6-n-propoxy-, 5-Brom-6-iso-propoxy-, 5-Brom-6-n-butoxy-, 5-Brom-6-sek.-butoxy-, 5-Brom-6-iso-butoxy- und 5-Brom-6-tert.-butoxy-2-cyclopropyl-4-hydroxy-pyrimidin, ferner 5-Methyl-6-methoxy-, 5-Methyl-6-äthoxy-, 5-Methyl-6-n-propoxy-, 5-Methyl-6-iso-propoxy-, 5-Methyl-6-n-butoxy-, 5-Methyl-6-sek.-butoxy-, 5-Methyl-6-iso-butoxy- und 5-Methyl-6-tert.-butoxy-2-cyclopropyl-4-hydroxy-pyrimidin, ferner 5-Äthyl-6-methoxy-, 5-Äthyl-6-äthoxy-, 5-Äthyl-6-n-propoxy-, 5-Äthyl-6-iso-propoxy-, 5-Äthyl-6-n-butoxy-, 5-Äthyl-6-sek.-butoxy-, 5-Äthyl-6-iso-butoxy- und 5-Äthyl-6-tert.-butoxy-2-cyclopropyl-4-hydroxy-pyrimidin.

Das Verfahren zur Herstellung der erfindungsgemäßen Verbindungen wird bevorzugt unter Mitverwendung geeigneter Lösungs- oder Verdünnungsmittel durchgeführt. Als solche kommen praktisch alle inerten organischen Solventien infrage. Hierzu gehören insbesondere aliphatische und aromatische, gegebenenfalls chlorierte Kohlenwasserstoffe, wie Benzol, Toluol, Xylol, Benzin, Methylenchlorid, Chloroform, Tetrachlorkohlenstoff, Chlorbenzol, oder Äther, z.B. Diäthyl- und Dibutyläther, Dioxan, ferner Ketone, beispielsweise Aceton, Methyläthyl-, Methylisopropyl- und Methylisobutylketon, außerdem Nitrile, wie Aceto- und Propionitril.

Als Säureakzeptoren können alle üblichen Säurebindemittel Verwendung finden. Besonders bewährt haben sich Alkalicarbonate und -alkoholate, wie Natrium- und Kaliumcarbonat, -methylat bzw. -äthylat, ferner aliphatische, aromatische oder heterocyclische Amine, beispielsweise Triäthylamin, Trimethylamin, Dimethylanilin, Dimethylbenzylamin und Pyridin.

Die Reaktionstemperatur kann innerhalb eines größeren Bereichs variiert werden. Im

allgemeinen arbeitet man zwischen 0 und 100°C, vorzugsweise bei 20 bis 60°C.

Die Umsetzung läßt man im allgemeinen bei Normaldruck ablaufen.

Zur Durchführung des Verfahrens setzt man die Ausgangsmaterialien meist in äquivalentem Verhältnis ein. Ein Überschuß der einen oder anderen Komponente bringt keine wesentlichen Vorteile. Die Reaktionspartner werden meist in einem der oben angegebenen Lösungsmittel in Gegenwart eines Säureakzeptors vereinigt und bei erhöhter Temperatur zur Vervollständigung der Reaktion eine oder mehrere Stunden gerührt. Danach versetzt man die Mischung mit einem organischen Lösungsmittel, z.B. Toluol, und arbeitet die organische Phase in üblicher Weise durch Waschen, Trocknen und Abdestillieren des Lösungsmittels auf.

Die neuen Verbindungen fallen oft in Form von Ölen an, die sich meist nicht unzersetzt destillieren lassen, jedoch durch sogenanntes "Andestillieren", d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex. Einige Verbindungen fallen in kristalliner Form an und werden durch ihren Schmelzpunkt charakterisiert.

Wie bereits mehrfach erwähnt, zeichnen sich die erfindungsgemäßen cyclopropylsubstituierten Pyrimidin(4)yl(thiono)(thiol)-phosphor(phosphon)-säureester bzw. -esteramide durch eine hervorragende insektizide und akarizide Wirkung aus. Sie wirken gegen Pflanzen-, Hygiene- und Vorratsschädlinge und auf dem veterinär-medizinischen Sektor. Sie besitzen bei geringer Phytotoxizität sowohl eine gute Wirkung gegen saugende als auch fressende Insekten und Milben.

Aus diesem Grunde können die erfindungsgemäßen Verbindungen mit Erfolg im Pflanzenschutz sowie auf dem Hygiene-, Vorratsschutz- und Veterinärsektor als Schädlingsbekämpfungsmittel eingesetzt werden.

Die Wirkstoffe eignen sich bei guter Pflanzenverträglichkeit und günstiger Warmblütertoxizität zur Bekämpfung von tierischen Schädlingen, insbesondere Insekten und Spinntieren, die in der Landwirtschaft, in Forsten, im Vorrats- und Material-schutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aëdes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmitteln können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; also feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion.

BEISPIEL A
Phaedon-Larven-Test

Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit der Wirkstoffzubereitung besprüht man Kohlblätter (Brassica oleracea) tropfnaß und besetzt sie mit Meerrettichblattkäfer-Larven (Phaedon cochleariae).

Nach bestimmten Zeiten wird die Abtötung in % bestimmt. In diesem Test zeigten z.B. die Verbindungen der Beispiele 1, 3, 6, 9 und 14 eine ausgezeichnete Wirkung, die der Wirkung der aus dem Stand der Technik bekannten Verbindungen deutlich überlegen ist.

BEISPIEL B
Tetranychus-Test (resistent)

Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit der Wirkstoffzubereitung werden Bohnenpflanzen (Phaseolus vulgaris), die stark von allen Entwicklungsstadien der gemeinen Spinnmilbe oder Bohnenspinnmilbe (Tetranychus urticae) befallen sind, tropfnaß besprüht.

Nach bestimmten Zeiten wird die Abtötung in % bestimmt. In diesem Test zeigen z.B. die Verbindungen der Beispiele 2, 3, 4 und 5 eine ausgezeichnete Wirkung, die derjenigen der aus dem Stand der Technik bekannten Verbindungen deutlich überlegen ist.

BEISPIEL C

$LD_{100}$-Test

Testtiere: Sitophilus granarius
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden boch. Anschließend gibt man etwa 25 Testtiere in die Petrischale und bedeckt sie mit einem Glasdeckel.

Der Zustand der Testtiere wird 3 Tage nach Ansetzen der Versuche kontrolliert. Bestimmt wird die Abtötung in %.

In diesem Test zeigen z.B. die Verbindungen der Beispiele 1, 2, 3 und 4 eine ausgezeichnete Wirkung, die derjenigen der aus dem Stand der Technik bekannten Verbindungen deutlich überlegen ist.

BEISPIEL D
Test mit parasitierenden Fliegenlarven

Emulgator: 80 Gewichtsteile Cremophor EL

Zur Herstellung einer zweckmäßigen Wirkstoffzubereitung vermischt man 20 Gewichtsteile der betreffenden aktiven Substanz mit der angegebenen Menge des Emulgators und verdünnt das so erhaltene Gemisch mit Wasser auf die gewünschte Konzentration.

Etwa 20 Fliegenlarven (Lucilia cuprina) werden in ein mit Wattestopfen entsprechender Größe beschicktes Teströhrchen gebracht, welches ca. 3 ml einer 20%igen Eigelbpulver-Suspension in Wasser enthält. Auf diese Eigelbpulver-Suspension werden 0,5 ml der Wirkstoffzubereitung gebracht. Nach 24 Stunden wird der Abtötungsgrad in % bestimmt.

In diesem Test zeigten z.B. die Verbindungen die Beispiele 1 bis 7, 9 und 11 eine ausgezeichnete Wirkung.

BEISPIEL E

Test mit parasitierenden adulten Rinderzecken (Boophilus microplus res.)

Lösungsmittel: Cremophor

Zur Herstellung einer zweckmäßigen Wirkstoffzubereitung vermischt man die betreffende aktive Substanz mit dem angegebenen Lösungsmittel im Verhältnis 1:2 und verdünnt das so erhaltene Konzentrat mit Wasser auf die gewünschte Konzentration.

10 adulte Rinderzecken (B. microplus res.) werden in der zu testenden Wirkstoffzubereitung 1 Min. getaucht. Nach Überführung in Plastikbecher und Aufbewahrung in einem klimatisierten Raum wird der Abtötungsgrad in Prozent bestimmt.

In diesem Test zeigt z.B. die Verbindung des Beispiels 4 eine ausgezeichnete Wirkung.

Herstellungsbeispiele

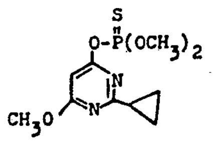

BEISPIEL 1

Eine Mischung aus 16,6 g (0,1 Mol) 2-Cyclopropyl-4-hydroxy-6-methoxy-pyrimidin, 20,7 g (0,15 Mol) Kaliumcarbonat, 16 g (0,1 Mol) O,O-Dimethylthionophosphorsäurediesterchlorid und 300 ml Acetonitril wird 4 Stunden bei 50°C gerührt. Dann kühlt man den Ansatz auf Raumtemperatur ab und schüttelt ihn nach Zugabe von 400 ml Toluol zweimal mit je 300 ml Wasser aus. Die organische Phase wird abgetrennt, über Natriumsulfat getrocknet, im Vakuum vom Lösungsmittel befreit und der Rückstand andestilliert. Man erhält so 22,6 g (78% der Theorie) O,O-Dimethyl-O-[2-cyclopropyl-6-methoxy-pyrimidin(4)yl]-thionophosphorsäureester in Form eines gelben Öles mit dem Brechungsindex $n_D^{23}$: 1,5441.

Analog Beispiel 1 können die folgenden Verbindungen der Formel

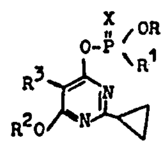

(I)

hergestellt werden:

| Beispiel Nr. | | $R^1$ | $R^2$ | $R^3$ | X | Ausbeute (% der Theorie) | Physikal. Daten (Brechungsindex; Schmelzpunkt °C) |
|---|---|---|---|---|---|---|---|
| 2 | $CH_3$ | $CH_3$ | $CH_3$ | H | S | 78 | $n_D^{23}$ : 1,5310 |
| 3 | $C_2H_5$ | $OC_2H_5$ | $CH_3$ | H | S | 95 | $n_D^{24}$ : 1,5166 |
| 4 | $C_2H_5$ | $SC_3H_7$-n | $CH_3$ | H | S | 73 | $n_D^{27}$ : 1,5449 |
| 5 | $C_2H_5$ | $CH_3$ | $CH_3$ | H | S | 76 | $n_D^{27}$ : 1,5343 |
| 6 | $C_3H_7$-n | $OC_2H_5$ | $CH_3$ | H | S | 72 | $n_D^{27}$ : 1,5127 |
| 7 | $C_2H_5$ | NH–$C_3H_7$-iso | $CH_3$ | H | S | 60 | 46 |
| 8 | $C_2H_5$ | $OC_2H_5$ | $CH_3$ | Br | S | 52 | $n_D^{23}$ : 1,5370 |
| 9 | $C_2H_5$ | $OC_2H_5$ | $CH_3$ | H | O | 83 | $n_D^{24}$ : 1,4886 |
| 10 | $C_2H_5$ | $OC_2H_5$ | $CH_3$ | $CH_3$ | S | 34 | $n_D^{25}$ : 1,5170 |
| 11 | $C_2H_5$ | NH–$C_3H_7$-iso | $CH_3$ | H | O | 53 | 44 |
| 12 | $CH_3$ | NH–$CH_3$ | $CH_3$ | H | S | 33 | $n_D^{25}$ : 1,5402 |
| 13 | $CH_3$ | $OCH_3$ | $C_2H_5$ | H | S | 70 | $n_D^{25}$ : 1,5254 |
| 14 | $C_2H_5$ | $OC_2H_5$ | $C_2H_5$ | H | S | 81 | $n_D^{25}$ : 1,5130 |
| 15 | $C_2H_5$ | $OC_2H_5$ | $CH_3$ | Cl | S | | |
| 16 | $C_2H_5$ | 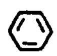 | $CH_3$ | H | S | | |
| 17 | $C_3H_7$-iso | $CH_3$ | $CH_3$ | H | S | | |
| 18 | $C_2H_5$ | $C_2H_5$ | $CH_3$ | H | S | | |
| 19 | $C_2H_5$ | $OC_2H_5$ | $CH_3$ | $C_2H_5$ | S | | |
| 20 | $C_2H_5$ | $OC_2H_5$ | $C_2H_5$ | $C_2H_5$ | S | | |
| 21 | $C_2H_5$ | $OC_2H_5$ | $C_3H_7$-n | H | S | | |
| 22 | $C_2H_5$ | $OC_2H_5$ | $C_3H_7$-iso | H | S | | |
| 23 | $CH_3$ | $OCH_3$ | $C_3H_7$-iso | H | S | | |
| 24 | $C_2H_5$ | $SC_3H_7$-n | $CH_3$ | H | O | 57 | $n_D^{23}$ : 1,5247 |
| 25 | $C_2H_5$ | $SC_3H_7$-n | $C_2H_5$ | H | S | 78 | $n_D^{25}$ : 1,5425 |
| 26 | $C_2H_5$ | $SC_3H_7$-n | $C_2H_5$ | H | O | | |

Die als Ausgangsmaterialien zu verwendenden 2-Cyclopropyl-4-hydroxy-pyrimidine können z.B. wie folgt hergestellt werden:

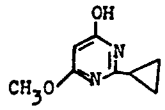

BEISPIEL a

Zu einer Lösung von 76 g (0,5 Mol) 2-Cyclopropyl-4,6-dihydroxypyrimidin in 250 ml 2 n Natronlauge tropft man bei 50°C 76 g (0,6 Mol) Dimethylsulfat. Durch gleichzeitige Zugabe von 2 n Natronlauge wird der pH-Wert der Reaktionslösung auf 8 bis 8,2 gehalten. Dann wird 2 Stunden unter weiterer pH-Kontrolle bei 50°C nachgerührt. Anschließend kühlt man das Gemisch auf 0°C ab und saugt das ausgefallene Produkt ab. Man erhält auf diese Weise 33 g (40% der Theorie) 2-Cyclopropyl-4-hydroxy-6-methoxypyrimidin in Form farbloser Kristalle mit dem Schmelzpunkt 186°C.

In analoger Weise können die folgenden Verbindungen der Formel

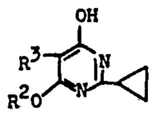 (III)

hergestellt werden:

| Beispiel | $R^2$ | $R^3$ | Ausbeute (% der Theorie) | Schmelzpunkt °C |
|---|---|---|---|---|
| b | $CH_3$ | $CH_3$ | 73 | 199 |
| c | $C_2H_5$ | H | 62 | 158 |
| d | $CH_3$ | Cl | | |
| e | $CH_3$ | $C_2H_5$ | | |
| f | $C_3H_7$-n | H | | |
| g | $C_3H_7$-iso | H | | |
| h | $C_2H_5$ | $C_2H_5$ | | |

BEISPIEL i

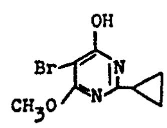

Eine Lösung von 12,4 g (0,075 Mol) 2-Cyclopropyl-4-hydroxy-6-methoxypyrimidin in 100 ml Methylenchlorid wird bei Raumtemperatur mit 12 g (0,075 Mol) Brom versetzt. Man rührt das Gemisch 1 Stunde bei Raumtemperatur nach, wäscht es dann mit 100 ml 2,5%iger Natriumhydrogencarbonatlösung und mit 100 ml Wasser und trocknet die organische Phase über Natriumsulfat. Nach Abdestillieren des Lösungsmittels bleiben 6,7 g (36% der Theorie) 2-Cyclopropyl-4-hydroxy-5-brom-6-methoxy-pyrimidin als farbloses Pulver mit dem Schmelzpunkt 174°C (Z) zurück.

**Patentansprüche**

1. Verbindungen der Formel

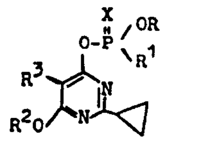 (I)

in welcher
R für Alkyl,
$R^1$ für Alkyl, Alkoxy, Alkylthio, Monoalkylamino oder Phenyl
$R^2$ für Alkyl,
$R^3$ für Wasserstoff, Halogen oder Alkyl und
X für Sauerstoff oder Schwefel stehen.

2. Verfahren zur Herstellung von Verbindungen der Formel (I), dadurch gekennzeichnet, daß man (Thiono)-(Thiol)phosphor(phosphon)-säureester- bzw. -esteramidhalogenide der Formel

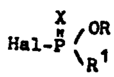 (II)

in welcher
R, $R^1$ und X die in Anspruch 1 angegebene Bedeutung haben und
Hal für Halogen, vorzugsweise Chlor, steht,
mit 2-Cyclopropyl-4-hydroxy-pyrimidinen der Formel

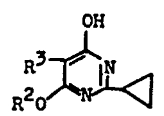 (III)

in welcher
$R^2$ und $R^3$ die in Anspruch 1 angegebene Bedeutung haben, gegebenenfalls in Gegenwart eines Säureakzeptors oder gegebenenfalls in Form der Alkali-, Erdalkali- oder Ammoniumsalze und gegebenenfalls in Gegenwart eines Lösungs- oder Verdünnungsmittels umsetzt.

3. Insektizide und akarizide Mittel, gekennzeichnet durch einen Gehalt an Verbindungen gemäß Anspruch 1.

4. Verwendung von Verbindungen gemäß Anspruch 1 zur Bekämpfung von Insekten und Spinntieren.

5. Verfahren zur Herstellung von insektiziden und akariziden Mitteln, dadurch gekennzeichnet, daß man Verbindungen gemäß Anspruch 1 mit Streckmitteln und/oder oberflächenaktiven Mitteln mischt.

**Revendications**

1. Composés, caractérisés en ce qu'ils répondent à la formule:

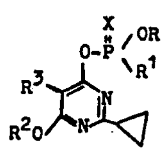 (I)

dans laquelle:
R est un groupe alkyle,
$R^1$ est un groupe alkyle, alkoxy, alkylthio, mono-alkylamino ou phényle,
$R^2$ est un groupe alkyle,
$R^3$ est un atome d'hydrogène ou d'halogène ou un groupe alkyl et
X est un atome d'oxygène ou de soufre.

2. Procédé de production de composés de formule (I), caractérisé en ce qu'il consiste à faire réagir des halogénures d'esters ou d'amides d'esters d'acide(thiono)-(thiol)-phosphorique(phosphonique) de formule:

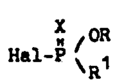 (II)

(dans laquelle R, $R^1$ et X ont les définitions données ci-dessus et Hal est un halogène, de préférence le chlore) avec des 2-cyclopropyl-4-hydroxypyrimidines de formule:

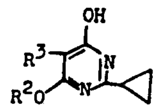

(III)

(dans laquelle $R^2$ et $R^3$ ont les définitions données dans la revendication 1), éventuellement en présence d'un accepteur d'acide ou, le cas échéant, sous la forme des sels de métaux alcalins ou alcalino-terreux ou d'ammonium et en la présence éventuelle d'un solvant ou d'un diluant.

3. Compositions insecticides et acaricides, caractérisées par une teneur en composés suivant la revendication 1.

4. Utilisation de composés suivant la revendication 1 pour la lutte contre des insectes et des acariens.

5. Procédé de production de compositions insecticides et acaricides, caractérisé en ce qu'il consiste à mélanger des composés suivant la revendication 1 avec des diluants et/ou des agents tensio-actifs.

## Claims

1. Compounds of the formula

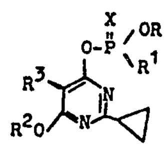

(I)

in which

R represents alkyl,

$R^1$ represents alkyl, alkoxy, alkylthio, monoalkylamino or phenyl,

$R^2$ represents alkyl,

$R^3$ represents hydrogen, halogen or alkyl and

X represents oxygen or sulphur.

2. A process for the preparation of compounds of formula (I) characterised in that (thiono)(thiol)-phosphoric(phosphonic) acid ester halides or ester-amide halides of the formula

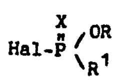

(II),

in which

R, $R^1$ and X have the meaning stated in claim 1 and

Hal represents halogen, preferably chlorine,

are reacted with 2-cyclopropyl-4-hydroxy-pyrimidines of the formula

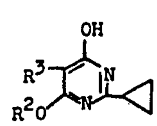

(III),

in which

$R^2$ and $R^3$ have the meaning stated in claim 1, optionally in the presence of an acid acceptor or optionally in the form of the alkali metal salts, alkaline earth metal salts of ammonium salts and optionally in the presence of a solvent or a diluent.

3. Insecticides and acaricides, characterised in that they contain compounds according to claim 1.

4. The use of compounds according to claim 1 for combating insects and Arachnida.

5. A process for the preparation of insecticides and acaricides, characterised in that compounds according to claim 1 are mixed with extenders and/or surface-active agents.